(19) **Europäisches Patentamt**
**European Patent Office**
**Office européen des brevets**

(11) **EP 4 353 219 A1**

(12) **EUROPEAN PATENT APPLICATION**
published in accordance with Art. 153(4) EPC

(43) Date of publication:
**17.04.2024 Bulletin 2024/16**

(21) Application number: **22820129.9**

(22) Date of filing: **02.06.2022**

(51) International Patent Classification (IPC):
*A61K 8/87* (2006.01)      *A61K 8/35* (2006.01)
*A61K 8/37* (2006.01)      *A61K 8/46* (2006.01)
*A61K 8/55* (2006.01)      *A61K 8/81* (2006.01)
*A61Q 3/02* (2006.01)

(52) Cooperative Patent Classification (CPC):
**A61K 8/35; A61K 8/37; A61K 8/46; A61K 8/55;**
**A61K 8/81; A61K 8/87; A61Q 3/02**

(86) International application number:
**PCT/JP2022/022432**

(87) International publication number:
**WO 2022/259947 (15.12.2022 Gazette 2022/50)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **08.06.2021 JP 2021095549**

(71) Applicant: **ThreeBond Co., Ltd.**
**Tokyo 192-0398 (JP)**

(72) Inventor: **MORIKAWA, Yumi**
**Hachioji-shi, Tokyo 192-0398 (JP)**

(74) Representative: **Petty, Catrin Helen et al**
**Venner Shipley LLP**
**200 Aldersgate**
**London EC1A 4HD (GB)**

(54) **PHOTOCURABLE RESIN COMPOSITION FOR NAIL OR ARTIFICIAL NAIL**

(57)      Provided is a photocurable resin composition for a nail or an artificial nail with which a cured product having gloss can be formed. A photocurable resin composition for a nail or an artificial nail including components (A) to (D) below, in which 0.01 parts by mass or more and 3.0 parts by mass or less of the component (C) with respect to 100 parts by mass of a compound having a (meth)acryloyl group is contained: the component (A): a urethane (meth)acrylate oligomer; the component (B): a compound having three or more functional groups which are (meth)acryloyl groups (excluding the component (A)); the component (C): a polyfunctional thiol compound; and the component (D): a photoinitiator.

EP 4 353 219 A1

**Description**

**TECHNICAL FIELD**

The present invention relates to a photocurable resin composition suitable for coating of a nail or an artificial nail.

**BACKGROUND ART**

In the related art, a photocurable resin composition (UV gel nail) containing a photopolymerizable monomer and/or oligomer is known in the field of nail art. In these UV gel nails, a resin is applied to a nail using a brush or the like and is irradiated with light and cured to decorate the nail and apply a cosmetic, so that a nail cosmetic film having beautiful gloss and high adhesion to the nail can be obtained. In this UV gel nail, multiple layers of a base coat layer, a color layer, and a topcoat layer are formed, and in particular, in the topcoat layer, a colorless and transparent appearance is most important together with gloss in order to make a base decoration look beautiful, and hardness for protecting the decoration and coating is also required.

In JP 2019-6689 A, an artificial nail composition excellent in surface glossiness including a urethane (meth)acrylate oligomer, a polyfunctional thiol compound having two or more thiol groups in one molecule, and a radically polymerizable compound having one or more radically polymerizable unsaturated bonds in one molecule is disclosed.

In JP 2017-210475 A, a glossy photocurable artificial nail composition including a urethane (meth)acrylate oligomer, a (meth)acrylic monomer, a polyfunctional thiol, and a photopolymerization initiator is disclosed.

**SUMMARY OF INVENTION**

However, it was difficult for a cured product of the compositions described in JP 2019-6689 A and JP 2017-210475 A to satisfy having high glossiness.

The present invention has been made in view of the above circumstances, and an object thereof is to provide a photocurable resin composition for a nail or an artificial nail with which a cured product having excellent gloss can be formed.

Solution to Problem

The gist of the present invention will be described below.

[1] A photocurable resin composition for a nail or an artificial nail including components (A) to (D) below, in which 0.01 parts by mass or more and 3 (3.0) parts by mass or less of the component (C) with respect to 100 parts by mass of a compound having a (meth)acryloyl group or a compound having a (meth)acryloyl group including the component (A) and the component (B) is contained:

> the component (A): a urethane (meth)acrylate oligomer;
> the component (B): a compound having three or more functional groups which are (meth)acryloyl groups (excluding the component (A));
> the component (C) is a polyfunctional thiol compound; and
> the component (D) is a photoinitiator.

[2] The photocurable resin composition for a nail or an artificial nail according to [1], in which the component (C) is a polyfunctional thiol compound having a trimethylolpropane skeleton.

[3] The photocurable resin composition for a nail or an artificial nail according to [1] or [2], in which the component (B) is a compound having three functional groups which are (meth)acryloyl groups.

[4] The photocurable resin composition for a nail or an artificial nail according to any one of [1] to [3], further including a polyether-based plasticizer as a component (F).

[5] The photocurable resin composition for a nail or an artificial nail according to any one of [1] to [4], further including, as a component (E), a (meth)acryloyl group other than the component (A) and the component (B).

[6] The photocurable resin composition for a nail or an artificial nail according to any one of [1] to [5], in which a glossiness of a surface of a cured product when the photocurable resin composition for a nail or an artificial nail is applied so as to have a film thickness of 0.1 mm and cured at an integrated light quantity of 7.5 kJ/m$^2$ and an uncured product on the surface of the cured product is wiped off with a solvent is 67 or more and 88 or less at 20°.

[7] The photocurable resin composition for a nail or an artificial nail according to any one of [1] to [6], in which the photocurable resin composition for a nail or an artificial nail is for forming a topcoat layer.

[8] A cured product obtained by curing the photocurable resin composition for a nail or an artificial nail according to any one of [1] to [6].

[9] A method for coating a nail or an artificial nail, including applying the photocurable resin composition for a nail or an artificial nail according to any one of [1] to [6] to a nail or an artificial nail to form a coating film, and then irradiating the coating film with an energy ray to cure the coating film.

## DESCRIPTION OF EMBODIMENTS

Details of the invention will be described below. In the present specification, "X to Y" is used to mean that numerical values (X and Y) stated before and after the "X to Y" are included as a lower limit value and an upper limit value. In addition, unless otherwise specified, operations and measurements of physical properties and the like are performed under conditions of room temperature (20°C to 25°C)/relative humidity of 40% to 50% RH.

An aspect of the present invention is a photocurable resin composition for a nail or an artificial nail including components (A) to (D) below, in which 0.01 parts by mass or more and 3.0 parts by mass or less of the component (C) with respect to 100 parts by mass of a compound having a (meth)acryloyl group is contained:

the component (A): a urethane (meth)acrylate oligomer;
the component (B): a compound having three or more functional groups which are (meth)acryloyl groups (excluding the component (A));
the component (C): a polyfunctional thiol compound; and
the component (D): a photoinitiator.

According to the photocurable resin composition for a nail or an artificial nail according to an aspect of the present invention, a cured product having excellent glossiness can be formed.

<Component (A)>

The component (A) included in the photocurable resin composition according to the present invention is a urethane (meth)acrylate oligomer, and as the component (A), any oligomer having one or more urethane bonds and one or more (meth)acryloyl groups can be used. When the urethane (meth)acrylate oligomer is added, effects of improving adhesion to a nail or an artificial nail and improving curability and strength of the photocurable resin composition (coating film) are obtained. In addition, the term "oligomer" refers to a polymer in which monomer units (including a monomer unit other than (meth)acrylate monomer) are repeated about 2 to several tens of times. In the present specification, the compound having a (meth)acryloyl group refers to a (meth)acrylate. The (meth)acryloyl group may have a (meth)acryloyl group as a form of a (meth)acryloyloxy group. Also, the term "(meth)acryloyl" encompasses both acryloyl and methacryloyl. Thus, for example, the term "(meth)acryloyl group" encompasses both acryloyl group ($H_2C=CH-C(=O)-$) and methacryloyl group ($H_2C=C(CH_3)-C(=O)-$). In addition, similarly, the term "(meth)acrylate" encompasses both acrylate and methacrylate, and the term "(meth)acrylic" encompasses both acrylic and methacrylic.

The component (A) is not particularly limited as long as the component is an oligomer having one or more urethane bonds and (meth)acryloyl groups, but preferably has two to six (meth)acryloyl groups, more preferably has two or three (meth)acryloyl groups, and most preferably has three (meth)acryloyl groups. The component (A) most preferably has two (meth)acryloyl groups. In addition, the (meth)acryloyl group of the component (A) is preferably an acryloyl group. It is noted that other functional groups such as a carboxy group, a phosphate group, and a hydroxyl group may further be included in addition to the urethane bond and the (meth)acryloyl group.

A weight average molecular weight of the oligomer of the component (A) is preferably 1,000 to 100,000, more preferably 1,200 to 30,000, and particularly preferably 1,500 to 20,000. Within such a range, curability of the cured product can be improved while maintaining a viscosity with good workability. In the present specification, for the weight average molecular weight, a value measured by gel permeation chromatography (GPC) using polystyrene as a standard substance is used.

The component (A) can be synthesized by forming a urethane bond by a reaction between a polyol and a polyisocyanate and adding a compound having a hydroxyl group and a (meth) acryloyl group in a molecule or a (meth)acrylic acid to an unreacted isocyanate group, but a method for synthesizing the urethane (meth)acrylate oligomer is not limited to the method.

In particular, from the viewpoint of adhesion, it is preferable to use, as the polyol, a polyether skeleton urethane (meth)acrylate oligomer using a polyether polyol or a polyether polyol having an aromatic group such as bisphenol. The component (A) is preferably a polyether skeleton bifunctional urethane (meth)acrylate oligomer. However, for example, other urethane (meth)acrylate oligomers such as a polyester skeleton urethane (meth)acrylate oligomer, a polycaprolactone skeleton urethane (meth)acrylate oligomer, a polycarbonate skeleton urethane (meth)acrylate oligomer, and the like can also be used in combination.

Specific examples of a commercially available product include AH-600 and UA-510H (manufactured by Kyoeisha Chemical Co., Ltd.), SUA-008 and SUA-023 (manufactured by Asia Chemical Industry Co., Ltd.), UN-6060S, UN-6060PTM, UN-6200, UN-6207, UN-6303, UN-6304, UN-6305, and UN-6306 (manufactured by Negami Chemical Industrial Co., Ltd.), and the like, but are not limited thereto. The component (A) can be used alone or two or more kinds thereof can also be used in combination.

<Component (B)>

The component (B) included in the photocurable resin composition according to the present invention is a compound having three or more functional groups which are (meth)acryloyl groups (excluding the component (A)). The number of (meth)acryloyl groups contained in one molecule is not particularly limited as long as it is three or more functional groups, but is preferably three to eight functional groups, more preferably three to six functional groups, and most preferably three functional groups, for the purpose of obtaining a cured product having good hardness. In a preferred embodiment, the component (B) is a compound having three functional groups which are (meth)acryloyl groups.

Specific examples of the component (B) included in the photocurable resin composition according to the present invention include trimethylolpropane tri(meth)acrylate, trimethylolethane tri(meth)acrylate, ethoxylated trimethylolpropane (meth)acrylate, ethoxylated trimethylolpropane tri(meth)acrylate, isocyanuric acid EO-modified tri(meth)acrylate, pentaerythritol tri(meth)acrylate, EO-modified trimethylolpropane tri(meth)acrylate, PO-modified trimethylolpropane tri(meth)acrylate, ECH-modified trimethylolpropane tri(meth)acrylate, ECH-modified glycerol tri(meth)acrylate, tris(acryloyloxyethyl)isocyanurate, pentaerythritol tetra(meth)acrylate, tetramethylolmethane tetra(meth)acrylate, dipentaerythritol tetra(meth)acrylate, ditrimethylolpropane tetra(meth)acrylate, dipentaerythritol hexa(meth)acrylate, caprolactone-modified pentaerythritol hexa(meth)acrylate, dipentaerythritol penta(meth)acrylate, caprolactone-modified pentaerythritol penta(meth)acrylate, and the like. Among these, the ethoxylated trimethylolpropane tri(meth)acrylate, the isocyanuric acid EO-modified tri(meth)acrylate, and/or the trimethylolpropane tri(meth)acrylate are preferable from the viewpoint of obtaining moderate hardness and excellent glossiness. The component (B) can be used alone or two or more kinds thereof can also be used in combination.

In the present invention, the content of the component (B) is 3 to 50 parts by mass, more preferably 5 to 40 parts by mass, and still more preferably 10 to 30 parts by mass, with respect to 100 parts by mass of the component (A). In a case where the component (B) is 3 parts by mass or more, the hardness of the cured product can be maintained. On the other hand, in a case where (B) is 50 parts by mass or less, the transparency of the cured product can be maintained.

<Component (C)>

The component (C) included in the photocurable resin composition according to the present invention is a polyfunctional thiol compound. The content of the component (C) is, for example, 0.01 to 4.0 parts by mass, 0.01 to 3 (3.0) parts by mass, more preferably 0.1 to 2.8 parts by mass, and still more preferably 1 to 2.5 parts by mass, with respect to 100 parts by mass of the compound having a (meth)acryloyl group contained in the photocurable resin composition or the compound having a (meth)acryloyl group including the component (A) and the component (B). When the polyfunctional thiol compound is contained in the above range, a cured product having excellent gloss can be obtained. As the polyfunctional thiol compound, any compound having two or more thiol groups in one molecule can be used, but from the viewpoint of glossiness, the thiol groups are preferably three to six functional groups and most preferably three functional groups. In addition, regarding a functional group ratio between the (meth)acryloyl groups and the thiol groups, the number of the (meth)acryloyl groups/the number of the thiol groups is preferably 9 or more, and still more preferably 11 or more. In addition, although not particularly limited, as an upper limit value, the number of the (meth)acryloyl groups/the number of the thiol groups is 1000. The "100 parts by mass of the compound having a (meth)acryloyl group" means 100 parts by mass in consideration of a blending amount of a component (E) in a case of a composition including the component (E) to be described later, and the "functional group ratio between the (meth)acryloyl groups and the thiol groups" means a ratio in consideration of a (meth)acryloyl group of the component (E) in a case of the composition including the component (E) to be described later. In addition, the content of the component (C) is, for example, 0.01 to 4.0 parts by mass, preferably 0.1 to 3.9 parts by mass, and more preferably 1 to 3.8 parts by mass, with respect to 100 parts by mass of the component (A) and the component (B) in total.

The functional group ratio between the (meth)acryloyl groups and the thiol groups can be calculated from, for example, a (meth)acryloyl group equivalent and a thiol group equivalent obtained by the following method.

As a method for measuring the (meth)acryloyl group equivalent, the following method can be used.

1 to 2 g of the compound having (meth)acryloyl groups is dissolved in 20 ml of acetone, and 10 ml of a 20 mass% methanol solution of morpholine is added. Furthermore, 1 ml of a 75 mass% acetic acid aqueous solution is added to be reacted for 30 minutes. After completion of the reaction, 25 ml of a 40 mass% acetonitrile solution of acetic anhydride is added thereto and stirred, and then titrated with a 0.5 mol/L hydrogen chloride methanol solution (titration amount: A1). Titration is performed with a 0.5 mol/L hydrogen chloride methanol solution in the same manner as described above, except that a compound having a (meth)acryloyl group is not used, as a blank assumption (titration amount: A0). The (meth)acryloyl group equivalent is calculated from the following formula:

$$\text{(meth)acryloyl group equivalent (g/eq)} = 2000 \times S/[(A0 - A) \times f]$$

In the formula, S is the mass (g) of the compound having a (meth)acryloyl group, and f is the titer of a 0.5 mol/L hydrogen chloride methanol solution.

As a method for measuring the thiol group equivalent, the following method (iodine titration method) can be used.

20 mL of chloroform is added to 0.2 g of the polyfunctional thiol compound to prepare a sample solution. As a starch indicator, 0.275 g of soluble starch is dissolved in 30 g of pure water. 20 mL of pure water, 10 mL of isopropyl alcohol, and 1 mL of a starch indicator are added to the sample solution, and stirred with a stirrer. An iodine solution is added dropwise, and a point at which a chloroform layer becomes green is defined as an end point. The thiol group equivalent is calculated from the following formula:

$$\text{thiol group equivalent (g/eq)} = \text{mass (g) of polyfunctional thiol compound} \times 10,000/\text{titration amount of iodine solution (mL)} \times \text{factor of iodine solution.}$$

As the (meth)acryloyl group equivalent and the thiol group equivalent, equivalents calculated from chemical structures may be used.

Specific examples of the component (C) contained in the photocurable resin composition according to the present invention include 1,2-ethanedithiol, 1,2-propanedithiol, 1,3-propanedithiol, 1,3-butanedithiol, 2,3-butanedithiol, 1,5-pentanedithiol, 1,6-hexanedithiol, 1,8-octanedithiol, 1,9-nonanedithiol, 1,10-decanedithiol, 1,2-benzenedithiol, 1,3-benzenedithiol, 1,4-benzenedithiol, 3,6-dichloro-1,2-benzenedithiol, toluene-3,4-dithiol, 1,5-naphthalenedithiol, ethylene glycol bis(thioglycolate), ethylene glycol bis(3-mercaptopropionate), 1,4-butanediol bisthioglycolate, tetraethylene glycol bis(3-mercaptopropionate), trimethylolpropane tris(thioglycolate), trimethylolpropane tris(3-mercaptopropionate), trimethylolpropane tris(3-mercaptobutyrate), tris[(3-mercaptopropionyloxy)-ethyl]isocyanurate, pentaerythritol tetrakis(thioglycolate), pentaerythritol tetrakis(3-mercaptopropionate), dipentaerythritol hexakis(3-mercaptopropionate), 1,4-bis(3-mercaptobutyryloxy)butane, pentaerythritol tetrakis(3-mercaptobutyrate), pentaerythritol tetrakis(3-mercaptobutylate), 1,3,5-tris(3-mercaptobutyloxyethyl)-1,3,5-triazine-2,4,6(1H,3H,5H)-trione, dimercaptodiethylsulfide, 1,8-dimercapto-3,6-dithiaoctane, 1,2-bis[(2-mercaptoethyl)thio]-3-mercaptopropane, tetrakis(7-mercapto-2,5-dithiaheptyl)methane, trithiocyanuric acid, 1,2-benzenedimethane, thiol, 4,4'-thiobisbenzenethiol, 2-din-butylamino-4,6-dimercapto-s-triazine, 2-di-n-butylamino-4,6-dimercapto-s-triazine, 2,5-dimercapto-1,3,4-thiadiazole, 1,8-dimercapto-3,6-dioxaoctane, 1,5-dimercapto-3-thiapentane, tris(2-hydroxyethyl)isocyanurate trimercaptopropionate, 1,4-dimethylmercaptobenzene, 2,4,6-trimercapto-s-triazine, 2-(N,N-dibutylamino)-4,6-dimercapto-s-triazine, bis(4-(2-mercaptopropoxy)phenyl)methane, 1,1-bis(4-(2-mercaptopropoxy)phenyl)ethane, 2,2-bis(4-(2-mercaptopropoxy)phenyl)propane, 2,2-bis(4-(2-mercaptopropoxy)phenyl)butane, 1,1-bis(4-(2-mercaptopropoxy)phenyl)isobutane, 2,2-bis(4-(2-mercaptopropoxy)-3-methylphenyl)propane, 2,2-bis(4-(2-mercaptopropoxy)-5-methylphenyl)propane, bis(2-(2-mercaptopropoxy)-5-methylphenyl)methane, 2,2-bis(4-(2-mercaptopropoxy)-3-t-butylphenyl)propane, tris(4-(2-mercaptopropoxy)phenyl)methane, 1,1,1-tris(4-(2-mercaptopropoxy)phenyl)ethane, bis(4-(2-mercaptobutoxy)phenyl)methane, 2,2-bis(4-(2-mercaptobutoxy)phenyl)propane, tris(4-(2-mercaptobutoxy)phenyl)methane, 1,3,5-triazine-2,4,6-trithiol, and the like. Examples of the polyfunctional thiol compound preferably include polyfunctional thiol compounds having a trimethylolpropane skeleton, a pentaerythritol skeleton, and a dipentaerythritol skeleton, particularly preferably include trimethylolpropane tris(3-mercaptopropionate), trimethylolpropane tris(3-mercaptobutyrate), tris[(3-mercaptopropionyloxy)-ethyl]-isocyanurate, pentaerythritol tetrakis(3-mercaptopropionate), pentaerythritol tetrakis(3-mercaptobutyrate), dipentaerythritol hexakis(3-mercaptopropionate), and dipentaerythritol hexakis(3-mercaptobutyrate), further preferably include polyfunctional thiols having a trimethylolpropane skeleton, and most preferably include trimethylolpropane tris(3-mercaptopropionate) and trimethylolpropane tris(3-mercaptobutyrate). When having these skeletons, a cured product having more excellent glossiness can be obtained. In a preferred embodiment, the component (C) is a polyfunctional thiol compound having a trimethylolpropane skeleton. The component (C) can be used alone or two or more kinds thereof can also be used in combination.

<Component (D)>

The component (D) included in the photocurable resin composition according to the present invention is a photoinitiator. Examples of the component (D) include a radical-based photoinitiator that generates radical species by energy rays such as visible rays, ultraviolet rays, X-rays, and electron beams, a cationic photoinitiator that generates cationic species, and an anionic photogenerator that generates anionic species. However, among these, from the viewpoint of reactivity and the like, the radical-based photoinitiator is preferable, and it is preferable that a visible ray radical-based photoinitiator and an ultraviolet radical-based photoinitiator are used in combination. In addition, from the viewpoint of long-term storage stability, it is preferable that the ultraviolet radical-based photoinitiator and the visible ray radical-based photoinitiator are contained at a ratio (mass ratio) of 5:5 to 7:3.

Examples of the radical-based photoinitiator include an acetophenone-based radical initiator, a benzoin-based radical initiator, a benzophenone-based radical initiator, a thioxanthone-based radical initiator, an acylphosphine oxide-based radical initiator, and a titanocene-based radical initiator. Among these, the acetophenone-based radical initiator and the acylphosphine oxide-based radical initiator are preferable from the viewpoint of excellent photocurability. In addition, these may be used alone, or two or more kinds thereof may also be used in combination. The acetophenone-based radical initiator corresponds to the ultraviolet radical-based photoinitiator, and the acylphosphine oxide-based radical initiator corresponds to the visible ray radical-based photoinitiator.

Examples of the acetophenone-based radical initiator include diethoxyacetophenone, 2-hydroxy-2-methyl-1-phenylpropane-1-one, benzyl dimethyl ketal, 4-(2-hydroxyethoxy)phenyl-(2-hydroxy-2-propyl)ketone, 1-hydroxy-cyclohexyl-phenyl-ketone, 2-methyl-2-morpholino(4-thiomethylphenyl)propane-1-one, 2-benzyl-2-dimethylamino-1-(4-morpholinophenyl)butanone, 2-hydroxy-2-methyl-1-[4-(1-methylvinyl)phenyl]propanone oligomer, and the like. Examples of the acylphosphine oxide-based radical initiator include 2,4,6-trimethylbenzoyl-diphenyl-phosphine oxide and bis(2,4,6-trimethylbenzoyl)-phenylphosphine oxide. The component (D) can be used alone or two or more kinds thereof can also be used in combination. From the viewpoint of achieving both glossiness and curability, it is preferable that the acetophenone-based radical initiator and the acylphosphine oxide-based radical initiator are used in combination. Specifically, it is more preferable that the 1-hydroxy-cyclohexyl-phenyl-ketone and 2,4,6-trimethylbenzoyl-diphenyl-phosphine oxide are used in combination.

In the present invention, the content of the component (D) is, for example, 0.1 to 15 parts by mass, more preferably 1 to 12 parts by mass, and still more preferably 3 to 9 parts by mass, with respect to 100 parts by mass of the compound having a (meth)acryloyl group contained in the photocurable resin composition or the compound having a (meth)acryloyl group including the component (A) and the component (B). In a case where the component (D) is 0.1 parts by mass or more, the glossiness of the cured product can be maintained. On the other hand, in a case where the component (D) is 15 parts by mass or less, the transparency of the cured product can be maintained. The "100 parts by mass of the compound having a (meth)acryloyl group" means 100 parts by mass in consideration of a blending amount of the component (E) in a case of a composition including the component (E) to be described later. In addition, the content of the component (D) is preferably 3 to 10 parts by mass, with respect to 100 parts by mass of the component (A) and the component (B) in total.

<Optional component>

For the present invention, additives such as a compound having a (meth)acryloyl group other than the component (A) and the component (B), a filler, a conductive filler, a silane coupling agent, a plasticizer, a pressure-sensitive adhesive, an antifoaming agent, a pigment, a rust inhibitor, a leveling agent, a dispersant, a rheology modifier, a flame retardant, and the like can be used, within a scope not impairing the object of the present invention. In addition, it is preferable that the photocurable resin composition according to the present invention does not contain a solvent from the viewpoint of adhesion to a nail.

As the component (E), a compound having a (meth)acryloyl group other than the component (A) and the component (B) may be added to the photocurable resin composition according to the present invention. When adding the component (E), it is possible to perform improvement in the adhesion to the nail and improvement in fluidity of the photocurable resin composition. In a preferred embodiment, the photocurable resin composition according to the present invention further includes a compound having a (meth)acryloyl group other than the component (A) and the component (B), as the component (E). A mass ratio between the sum of the component (A) and the component (B) and the component (E) (component (A) + component (B):component (E)) is preferably 7:3 to 5:5.

Specific examples of the compound having one functional group which is a (meth)acryloyl group other than the component (A) and the component (B) include lauryl (meth)acrylate, stearyl (meth)acrylate, ethyl carbitol (meth)acrylate, tetrahydrofurfuryl (meth)acrylate, caprolactone-modified tetrahydrofurfuryl (meth)acrylate, cyclohexyl (meth)acrylate, dicyclopentanyl (meth)acrylate, isobornyl (meth)acrylate, benzyl (meth)acrylate, phenyl (meth)acrylate, phenoxyethyl (meth)acrylate, phenoxydiethylene glycol (meth)acrylate, phenoxytetraethylene glycol (meth)acrylate, nonylphenoxyphenoxyethyl (meth)acrylate, nonylphenoxytetraethylene glycol (meth)acrylate, methoxydiethylene glycol (meth)acrylate, ethoxydiethylene glycol (meth)acrylate, butoxyethyl (meth)acrylate, butoxytriethylene glycol (meth)acrylate, 2-ethylhexyl polyethylene glycol (meth)acrylate, 4-hydroxybutyl (meth)acrylate, nonylphenylpolypropylene glycol (meth)acrylate, methoxydipropylene glycol (meth)acrylate, 2-hydroxyethyl (meth)acrylate, 2-hydroxypropyl (meth)acrylate, (meth)acrylic acid, hydroxybutyl (meth)acrylate, 3-hydroxy-3-methylbutyl (meth)acrylate, hydroxypentyl (meth)acrylate, hydroxyhexyl (meth)acrylate, hydroxyheptyl (meth)acrylate, hydroxyoctyl (meth)acrylate, glycerol (meth)acrylate, polyethylene glycol (meth)acrylate, polypropylene glycol (meth)acrylate, epichlorohydrin-modified butyl (meth)acrylate, epichlorohydrin-modified phenoxy (meth)acrylate, N,N-dimethylaminoethyl (meth)acrylate, N,N-diethylaminoethyl (meth)acrylate, and the like, but are not limited thereto. Among these, it is preferable that the 2-hydroxyethyl (meth)acrylate, the 2-hydroxypropyl methacrylate and/or hydroxybutyl (meth)acrylate are added. Addition of these compounds makes it possible to lower the fluidity of the photocurable resin composition without lowering the reactivity.

Specific examples of the compound having two functional groups which are (meth)acryloyl groups other than the component (A) and the component (B) include 1,3-butylene glycol di(meth)acrylate, 1,4-butylene glycol di(meth)acrylate, tricyclodecane dimethanol di(meth)acrylate, neopentyl glycol di(meth)acrylate, 1,6-hexane glycol di(meth)acrylate, ethylene glycol diacrylate, tetraethylene glycol di(meth)acrylate, polyethylene glycol di(meth)acrylate, propylene glycol di(meth)acrylate, tripropylene glycol di(meth)acrylate, ethylene oxide-modified neopentyl glycol di(meth)acrylate, propylene oxide-modified neopentyl glycol di(meth)acrylate, bisphenol A ethoxylate di(meth)acrylate, ethylene oxide-modified bisphenol A di(meth)acrylate, epichlorohydrin-modified bisphenol A (meth)acrylate, ethylene oxide-modified bisphenol S di(meth)acrylate, neopentyl glycol-modified trimethylolpropane di(meth)acrylate, dicyclopentenyl di(meth)acrylate, ethylene oxide-modified dicyclopentenyl di(meth)acrylate, di(meth)acryloyl isocyanurate, isocyanuric acid EO-modified di(meth)acrylate, and the like, but are not limited thereto. Among these, it is preferable that the bisphenol A ethoxylate di(meth)acrylate and/or the isocyanuric acid EO-modified di(meth)acrylate are added. When adding these compounds, it is possible to improve the adhesion to the nail. The component (E) can be used alone or two or more kinds thereof can also be used in combination.

For the purpose of improving an elastic modulus, fluidity, and the like of a cured product, a filler may be added to the photocurable resin composition according to the present invention, within a scope not impairing the object of the present invention. Specific examples thereof include an inorganic powder, an organic powder, and the like.

Examples of the filler of the inorganic powder include glass, fumed silica, alumina, mica, ceramics, a silicone rubber powder, calcium carbonate, aluminum nitride, a carbon powder, kaolin clay, a dry clay mineral, dry diatomaceous earth, kaolin, and the like, but are not limited thereto. These can be used alone and two or more kinds thereof can also be used in combination. A blending amount of the inorganic powder is preferably about 0.1 to 200 parts by mass, with respect to 100 parts by mass of the component (A).

The fumed silica is blended for the purpose of adjusting the viscosity of the photocurable resin composition or improving the mechanical strength of the cured product. Preferably, fumed silica and the like which is surface-treated with dimethylsilane, trimethylsilane, alkylsilane, methacryloxysilane, organochlorosilane, polydimethylsiloxane, hexamethyldisilazane, or the like are used. Examples of a commercially available product of the fumed silica include AEROSIL R972, R972V, R972CF, R974, R976, R976S, R9200, RX50, NAX50, NX90, RX200, RX300, R812, R812S, R8200, RY50, NY50, RY200S, RY200, RY300, R104, R106, R202, R805, R816, T805, R711, R7200, and the like (manufactured by Nippon Aerosil Co., Ltd.), but are not limited thereto. These can be used alone and two or more kinds thereof can also be used in combination.

Examples of the filler of the organic powder include polyethylene, polypropylene, polystyrene, nylon, polyester, polyvinyl alcohol, polyvinyl butyral, polycarbonate, and polymethyl (meth)acrylate, but are not limited thereto. These can be used alone and two or more kinds thereof can also be used in combination. A blending amount of the organic powder is preferably about 0.1 to 200 parts by mass, with respect to 100 parts by mass of the component (A).

A conductive filler may be added to the photocurable resin composition according to the present invention. Examples of the conductive filler include gold, silver, platinum, nickel, palladium, and a plated particle in which an organic polymer particle is coated with a metal thin film, but are not limited thereto. These can be used alone and two or more kinds thereof can also be used in combination.

A silane coupling agent may be added to the photocurable resin composition according to the present invention. Examples of the silane coupling agent include γ-chloropropyl trimethoxysilane, octenyl trimethoxysilane, glycidoxyoctyl trimethoxysilane, β-(3,4-epoxycyclohexyl) ethyl trimethoxysilane, γ-glycidoxypropyl trimethoxysilane, γ-mercaptopropyl trimethoxysilane, γ-aminopropyl triethoxysilane, N-β-(aminoethyl)-γ-aminopropyl trimethoxysilane, N-β-(aminoethyl)-γ-aminopropyl methyldimethoxysilane, γ-ureidopropyl triethoxysilane, p-styryl trimethoxysilane, and the like, but are not limited thereto. These can be used alone and two or more kinds thereof can also be used in combination.

It is preferable that a plasticizer is added to the photocurable resin composition according to the present invention, as a component (F), from the viewpoint of the fluidity. The component (F) is not particularly limited, and examples thereof include, as the polyether-based plasticizer, a compound having a polyether skeleton. The polyether skeleton means a skeleton having an alkylene oxide such as polypropylene glycol, polyethylene glycol, polybutylene glycol, and the like.

The number of repetitions of the alkylene oxide is not particularly limited, and is, for example, 3 to 300, more preferably 5 to 100, and particularly preferably in the range of 10 to 60. In addition, examples of the polycarboxylic acid ester plasticizer include an aromatic polycarboxylic acid ester, phthalic acid ester including dioctyl phthalate (DOP), dibutyl phthalate (DBP), diheptyl phthalate (DHP), diisononyl phthalate (DINP), diisodecyl phthalate (DIDP), butyl benzyl phthalate (BBP), and the like, trimellitic acid ester including trioctyl trimellitic acid (TOTM), triisodecyl trimellitic acid (TITM), and the like, pyromellitic acid ester including tetraoctyl pyromellitic acid and the like, aliphatic polycarboxylic acid ester including di-2-ethylhexyl adipate (DOA), isodecyl adipate (DIDA), di-2-ethylhexyl sebacate (DOS), dibutyl sebacate (DBS), di-2-ethylhexyl maleate (DOM), dibutyl fumarate (DBF), di-2-ethylhexyl azelate (DOZ), di-2-ethylhexyl epoxyhexahydrophthalate, trioctyl citrate, glycerol triacetate, and the like, but are not limited thereto. In addition, examples of the phosphate ester-based plasticizer include trimethyl phosphate, tributyl phosphate, tri-(2-ethylhexyl) phosphate, tributoxyethyl phosphate, triphenyl phosphate, tricresyl phosphate, alkyl allyl phosphate, triethyl phosphate, tri(chloroethyl) phosphate, trisdichloropropyl phosphate, tris($\beta$-chloropropyl) phosphate, octyl diphenyl phosphate, tris(isopropylphenyl) phosphate, cresyl phenyl phosphate, and the like, but are not limited thereto. In addition, a plurality of plasticizers can be used in combination.

As the component (F), the polyether-based plasticizer is preferable, and the polypropylene glycol is more preferably used. When using the polyether-based plasticizer, the fluidity of a photocurable composition can be optimized without lowering the glossiness of the cured product. In a preferred embodiment, the photocurable resin composition according to the present invention further includes a polyether-based plasticizer as the component (F).

A number average molecular weight of the component (F) is not particularly limited, but is, for example, in the range of 200 to 30000, preferably in the range of 350 to 10000, and particularly preferably in the range of 500 to 5000. The number average molecular weight is calculated by a standard polystyrene conversion method using size exclusion chromatography (SEC) unless otherwise specified. Within the above range, it is possible to further obtain a radically curable thermally conductive resin composition capable of obtaining a cured product excellent in tensile strength and extensibility while maintaining thermal conductivity.

A commercially available product of the polyether-based plasticizer of the component (F) is not particularly limited, and examples thereof include PEG#300, PEG#400, PEG#600, PEG#1000, PEG#1500, PEG#15400, PEG#2000, PEG#4000, PEG#6000, PEG#1100, PEG#2000, UNIOL D-700, D-1000, D-1200, D-2000, D-4000, PB-500, PB-700, PB-1000, and PB-2000 (manufactured by NOF CORPORATION).

A blending amount of the component (F) is preferably 1 to 20 parts by mass, more preferably 3 to 15 parts by mass, and particularly preferably 5 to 13 parts by mass, with respect to 100 parts by mass of the compound having a (meth)acryloyl group contained in the photocurable resin composition or the compound having a (meth)acryloyl group including the component (A) and the component (B). When adding 1 part by mass or more of the component (F), the viscosity can be lowered, and when adding 20 parts by mass or less of the component (F), the glossiness of the cured product can be maintained. The "100 parts by mass of the compound having a (meth)acryloyl group" means 100 parts by mass in consideration of the blending amount of the component (E) in a case of a composition including the component (E). In addition, the content of the component (F) is preferably 5 to 18 parts by mass, with respect to 100 parts by mass of the component (A) and the component (B) in total.

As described above, according to the photocurable resin composition for a nail or an artificial nail according to the present invention, a cured product having excellent glossiness can be formed.

In one embodiment of the present invention, the glossiness of a surface of a cured product when the photocurable resin composition for a nail or an artificial nail according to the present invention is applied so as to have a film thickness of 0.1 mm and cured at an integrated light quantity of 7.5 kJ/m² and an uncured product on the surface of the cured product is wiped off with a solvent is 67 or more and 88 or less at 20°.

One embodiment of the present invention is a cured product obtained by curing the photocurable resin composition for a nail or an artificial nail according to the present invention.

The artificial nail in the present invention refers to a layer formed on a nail of human or animal for the purpose of decoration and/or protection. In addition, examples of other artificial nails described above include a resin base material (false nail) having a predetermined shape for the purpose of decorating and/or protecting the nail. In addition, the shape of the artificial nail is not particularly limited, and the artificial nail may be formed so as to cover the nail, or may be formed in a shape larger than the nail for the purpose of extending the nail. In addition, the artificial nail may be formed for the purpose of adhering an item such as a stone or the like to the nail for improving an aesthetic appearance.

Regarding a configuration of an artificial nail, generally, a base coat layer (a layer for the purpose of imparting adhesion to a nail, preventing color migration, and the like) formed by curing a photocurable resin composition for base coat is formed on a surface of a nail, a color layer (a layer containing a coloring material or the like for the purpose of decoration) formed by curing the photocurable resin composition for color is formed thereon, and a topcoat layer (a layer for the purpose of coating, imparting gloss, and improving an aesthetic appearance) formed by curing the photocurable resin composition for topcoat is further formed thereon. The photocurable resin composition of the present invention is not particularly limited as long as it is used for the purpose of forming a layer on a nail or an artificial nail, but it is preferably used as a photocurable resin composition for forming a topcoat layer because the photocurable resin composition has glossiness. In a preferred embodiment, the photocurable resin composition according to the present invention is for forming a topcoat layer.

In a case where the photocurable resin composition according to the present invention is applied directly to a nail before procedure, sanding is performed on the surface of the nail with a file (file) or the like in order to improve adhesion, and then dust, oil, moisture, and the like are removed with a nail dedicated solvent containing ethanol as a main component. When the photocurable resin composition according to the present invention is applied, a coating film having a thickness of 50 to 300 um is formed with a hair pencil, a brush, or the like, in a state before curing. In addition, the photocurable resin composition according to the present invention may be directly applied onto a cured film of a resin for a base coat, a resin for a color, or the like. When application is performed, a primer may be used in advance. When curing is performed, the photocurable composition is irradiated with an energy ray to be cured. The energy ray referred to herein includes all light in a broad sense such as radiation such as an $\alpha$ ray, a $\beta$ ray, and the like, an electromagnetic wave such as a $\gamma$ ray, an X ray, and the like, an electron beams(EB), an ultraviolet ray having a wavelength of about 100 to 380 nm, and a visible ray having a wavelength of about 381 to 800 nm, and is preferably the ultraviolet ray or the visible ray. As an irradiation device when curing is performed, a commercially available nail UV lamp or nail LED lamp is used. The irradiation time is 15 to 120 seconds, and is preferably 20 to 70 seconds in consideration of the influence on a finger. In addition, the integrated light quantity is preferably 3 to 15 kJ/m$^2$, and more preferably 5 to 10 kJ/m$^2$. In addition, in the photocurable resin composition of the present invention, it is preferable to wipe the surface of the cured product with a wiping solvent dedicated to nails. By doing so, more excellent gloss can be exhibited. Examples of the wiping solvent dedicated to nails include those containing ethanol, isopropyl alcohol, methyl ethyl ketone, acetone, or the like as a main component, but are not limited thereto.

Another aspect of the present invention is a method for coating a nail or an artificial nail, including applying the photocurable resin composition for a nail or a population nail according to the present invention to a nail or an artificial nail to form a coating film, and then irradiating the coating film with an energy ray to cure the coating film.

The photocurable resin composition of the present invention can be produced by a method known in the related art. For example, the photocurable resin composition can be produced by blending predetermined amounts of the components (A) to (D) and, if necessary, other components, and mixing the components at a temperature of preferably 10°C to 50°C for preferably 0.1 to 5 hours under a lightshielding condition using mixing means such as a planetary mixer or the like.

Examples

Hereinafter, the present invention will be described in more detail with reference to Examples, but the present invention is not limited to these Examples. In addition, hereinafter, the photocurable resin composition is also simply referred to as a resin.

<Preparation of Photocurable Resin Composition>

Each component was collected in parts by mass shown in Table 1, and mixed for 60 minutes in a 25°C environment under a light shielding condition using a planetary mixer to prepare a photocurable resin composition.

<Component (A)>

a1: Polyether skeleton bifunctional urethane acrylate (ART RESIN UN-6303, manufactured by Negami Chemical Industrial Co., Ltd., weight average molecular weight: 4000)

<Component (B)>

b1: Isocyanuric acid EO-modified triacrylate (Aronix M-313 (65 mass%), manufactured by Toagosei Co., Ltd.)
b2: Trimethylolpropane triacrylate (NK Ester A-TMPT, manufactured by Shin Nakamura Chemical Industry Co.)
b3: Trimethylolpropane trimethacrylate (NK Ester TMPT, manufactured by Shin Nakamura Chemical Industry Co.)
b4: Ethoxylated trimethylolpropane triacrylate (Sartomer SR502, manufactured by Sartomer)

<Component (C)>

c1: Trimethylolpropane tris(3-mercaptopropionate) (TMMP-20P, manufactured by SC Organic Chemical Co., Ltd.)
c2: trimethylolpropane tris(3-mercaptobutyrate) (Karenz MT TPMB, manufactured by Showa Denko K.K.)
c3: dipentaerythritol hexakis(3-mercaptopropionate) (DPMP, manufactured by SC Organic Chemical Co., Ltd.)
c4: Pentaerythritol tetrakis(3-mercaptopropionate) (PEMPII-20P, manufactured by SC Organic Chemical Co., Ltd.)
c5: Pentaerythritol tetrakis(3-mercaptobutyrate) (Karenz MT
PE1, manufactured by Showa Denko K.K.)

<Component (D)>

d1: 1-Hydroxy-cyclohexyl-phenyl-ketone (DOUBLECURE 184, manufactured by Double Bond Chemical Ind., Co., Ltd.)
d2: 2,4,6-Trimethylbenzoyl-diphenyl-phosphine oxide (DOUBLECURE TPO, manufactured by Double Bond Chemical Ind., Co., Ltd.)

<Component (E)>

e1: Isocyanuric acid EO-modified diiacrylate (Aronix M-313 (35 mass%), manufactured by Toagosei Co., Ltd.)
e2: Bisphenol A ethoxylate dimethacrylate (NK Ester BPE-80N, manufactured by Shin-Nakamura Chemical Co., Ltd.)
e3: 4-Hydroxybutyl acrylate (4-HBA, Osaka Organic Chemical Co., Ltd.)
e4: 2-Hydroxypropyl methacrylate (acrylic ester HP, Mitsubishi Rayon Co., Ltd.)

<Component (F)>

f1: Polypropylene glycol (UNIOL D-700, NOF CORPORATION).
Test methods used in Examples and Comparative Examples in Table 1 are as follows.

<Glossiness Measurement>

A surface of a black test piece (single-sided aminoalkyd clear after electrodeposition, material: SPCC-SD) having a size of 0.8 × 70 × 150 mm was degreased and washed, a spacer was set so that a film thickness was 0.1 mm, about 2 to 3 ml of a resin was applied, and squeegee was performed using a glass rod. After that, curing was performed using a nail LED lamp (LEXIA EX 30W wavelength 395 to 405 nm; manufactured by PREANFA CO., LTD.), a cured product for measurement was prepared (curing conditions: irradiation time 30 seconds and integrated light quantity 7.5 kJ/m$^2$). Thereafter, wipe was performed with cotton soaked with acetone. Values at 60° and 20° were measured using a gloss meter (Gloss Checker, manufactured by HORIBA, Ltd.). From the viewpoint of excellent gloss, ○ is preferable, and ⊙ is still more preferable in the following evaluation criteria.

<<Evaluation Criteria>>

⊙: 67 to 88 at 20°
○: 81 to 90 at 60°
×: 80 or less at 60°

<Viscosity Measurement>

0.5 mL of each of the photocurable resin compositions shown in Table 1 was collected and discharged into a measuring cup. Viscosity measurement was performed with an EHD viscometer (manufactured by Toki Sangyo Co., Ltd.) under the following conditions. The result was defined as "viscosity (Pa·s)". At the time of covering (decorating) a nail, from the viewpoint of workability such as suppression of resin flow, ease of application, and the like, ○ is preferable, and ⊙ is still more preferable in the following evaluation criteria.

<<Evaluation Criteria>>

⊙: 0.5 to 50 Pa·s
∘: 49 to 150 Pa·s
✕: less than 0.5 Pa·s or more than 150 Pa·s

<<Measurement conditions>>

Cone rotor: 3° ✕ R14
Rotation speed: 1 rpm
Measurement time: 3 minutes
Measurement temperature: 25°C (temperature was controlled by a chiller).

<Hardness Measurement>

A spacer having a thickness of 1 mm was placed on a blue plate glass having a size of 1.0 ✕ 150 ✕ 150 mm, and the photocurable resin composition was applied. A PET film was stacked thereon, and one sheet of another blue plate glass was further stacked thereon, and the photocurable resin composition was sandwiched therebetween, and irradiated twice from a front surface and a back surface of the two sheets of blue plate glass using a high-pressure mercury lamp (curing condition: integrated light quantity: 30 kJ/m$^2$) to prepare a cured product having a thickness of 1 mm (the curing was performed by adjusting UV transmitted through a PET film and blue plate glass to the above curing conditions). Three sheets of the cured product were prepared in the same manner and left for 2 hours. Thereafter, the blue plate glass and the PET film were peeled off, and three sheets of a sheet-like cured product having a thickness of 1 mm were stacked such that a surface to which the PET film was attached was directed upward. The hardness of the layered sheet-like cured product was measured on a smooth surface using a type D durometer tester. The measurement was performed five times, and an average value of three times excluding a maximum value and a minimum value was obtained. In daily life, in order to make a scratch and peeling less likely to occur, it is preferable to be ∘ in the following evaluation criteria.

<<Evaluation Criteria>>

∘: D70 to 100
✕: less than D70

<<Measurement conditions>>

Durometer pressing speed: 3.0 mm/sec
Numerical reading method: a maximum value within 1 second after the tip of a measuring unit of the durometer comes into close contact with the measurement object.

[Table 1]

| | Example 1 | Example 2 | Example 3 | Example 4 | Example 5 | Example 6 | Example 7 | Example 8 | Example 9 | Example 10 | Comparative Example 1 | Comparative Example 2 | Comparative Example 3 | Comparative Example 4 | Comparative Example 5 |
|---|---|---|---|---|---|---|---|---|---|---|---|---|---|---|---|
| a1 | 55.6 | 55.6 | 55.6 | 55.6 | 55.6 | 55.6 | 55.6 | 55.6 | 55.6 | 55.6 | 55.6 | 55.6 | 55.6 | 55.6 | 55.6 |
| b1 | 7.2 | 7.2 | 7.2 | 7.2 | 7.2 | 7.2 | | | | 7.2 | 7.2 | 7.2 | | | |
| b2 | | | | | | | 11.1 | | | | | | | | |
| b3 | | | | | | | | 11.1 | | | | | | | |
| b4 | | | | | | | | | 11.1 | | | | | | |
| e1 | 3.9 | 3.9 | 3.9 | 3.9 | 3.9 | 3.9 | | | | 3.9 | 3.9 | 3.9 | | | |
| e2 | 27.8 | 27.8 | 27.8 | 27.8 | 27.8 | 27.8 | 27.8 | 27.8 | 27.8 | 27.8 | 27.8 | 27.8 | 38.9 | 27.8 | 27.8 |
| e3 | 5.6 | | 5.6 | 5.6 | 5.6 | 5.6 | 5.6 | 5.6 | 5.6 | 5.6 | 5.6 | 5.6 | 5.6 | 16.7 | 5.6 |
| e4 | | 5.6 | | | | | | | | | | | | | |
| c1 | 2.3 | 2.3 | | | | | 2.3 | 2.3 | 2.3 | 2.3 | | 4.0 | 2.3 | 2.3 | 2.3 |
| c2 | | | 2.3 | | | | | | | | | | | | |
| c3 | | | | 2.3 | | | | | | | | | | | |
| c4 | | | | | 2.3 | | | | | | | | | | |
| c5 | | | | | | 2.3 | | | | | | | | | |
| d1 | 3.3 | 3.3 | 3.3 | 3.3 | 3.3 | 3.3 | 3.3 | 3.3 | 3.3 | 3.3 | 3.3 | 3.3 | 3.3 | 3.3 | 3.3 |
| d2 | 2.8 | 2.8 | 2.8 | 2.8 | 2.8 | 2.8 | 2.8 | 2.8 | 2.8 | 2.8 | 2.8 | 2.8 | 2.8 | 2.8 | 2.8 |
| f1 | 11.1 | 11.1 | 11.1 | 11.1 | 11.1 | 11.1 | 11.1 | 11.1 | 11.1 | | 11.1 | 11.1 | 11.1 | 11.1 | 11.1 |
| Total | 119.6 | 119.6 | 119.6 | 119.6 | 119.6 | 119.6 | 119.6 | 119.6 | 119.6 | 108.4 | 117.2 | 121.2 | 119.6 | 119.6 | 108.4 |
| Number of (meth) acryloyl groups/ Number of thiol groups | 14 | 14 | 16 | 14 | 18 | 15 | 17 | 16 | 13 | 14 | - | 8 | 13 | 15 | 11 |

(continued)

| | | Example 1 | Example 2 | Example 3 | Example 4 | Example 5 | Example 6 | Example 7 | Example 8 | Example 9 | Example 10 | Comparative Example 1 | Comparative Example 2 | Comparative Example 3 | Comparative Example 4 | Comparative Example 5 |
|---|---|---|---|---|---|---|---|---|---|---|---|---|---|---|---|---|
| Glossiness | Evaluation | ⊙ | ⊙ | ⊙ | ○ | ○ | ○ | ⊙ | ⊙ | ⊙ | ⊙ | × | × | × | × | × |
| | 60° | 87 | 84 | 86 | 82 | 82 | 85 | 82 | 82 | 83 | 88 | 80 | 77 | 74 | 68 | 70 |
| | 20° | 71 | 70 | 70 | 55 | 51 | 66 | 70 | 70 | 70 | 74 | 65 | 36 | 56 | 52 | 52 |
| Viscosity | | ⊙ | ⊙ | ⊙ | ⊙ | ⊙ | ⊙ | ⊙ | ⊙ | ⊙ | ○ | ⊙ | ⊙ | ⊙ | ⊙ | ⊙ |
| Hardness | | ○ | ○ | ○ | ○ | ○ | ○ | ○ | ○ | ○ | ○ | ○ | ○ | ○ | ○ | ○ |

According to Examples in Table 1, it can be seen that a photocurable resin composition can form a cured product with excellent glossiness. The component (C) is different between Example 1 and Examples 3 to 6, but every cured product has excellent glossiness, and the component (B) is different between Example 1 and Examples 7 to 9, but every cured product has excellent glossiness.

According to Comparative Examples in Table 1, since Comparative Example 1 does not include the component (C), the glossiness of the cured product is poor. In addition, in Comparative Example 2, since an added amount of the component (C) exceeds 3 parts by mass (3.0) with respect to 100 parts by mass of the component (A), the component (B), and the component (E), it can be seen that the glossiness of the cured product is poor. In addition, in Comparative Examples 3 and 4, the component (B) was replaced with the component (E), but a result that the glossiness of the cured product was not excellent was obtained. In addition, in Comparative Example 5, since the component (B) was not included, it can be seen that the glossiness of the cured product is poor.

Industrial Applicability

The photocurable resin composition of the present invention is a photocurable resin composition for a nail or an artificial nail which has hardness required for coating at the time of curing and can form a cured product with gloss, and therefore can be widely used in the field of nail art.

The present application is based on Japanese Patent Application No. 2021-095549 filed on June 8, 2021, the disclosure content of which is incorporated herein by reference in entirety thereof.

**Claims**

1. A photocurable resin composition for a nail or an artificial nail, comprising components (A) to (D) below, wherein 0.01 parts by mass or more and 3.0 parts by mass or less of the component (C) with respect to 100 parts by mass of a compound having a (meth)acryloyl group is contained:

   the component (A): a urethane (meth)acrylate oligomer;
   the component (B): a compound having three or more functional groups which are (meth)acryloyl groups (excluding the component (A)),
   the component (C): a polyfunctional thiol compound; and
   the component (D): a photoinitiator.

2. The photocurable resin composition for a nail or an artificial nail according to claim 1, wherein the component (C) is a polyfunctional thiol compound having a trimethylolpropane skeleton.

3. The photocurable resin composition for a nail or an artificial nail according to claim 1, wherein the component (B) is a compound having three functional groups which are (meth)acryloyl groups.

4. The photocurable resin composition for a nail or an artificial nail according to claim 1, further comprising a polyether-based plasticizer as a component (F).

5. The photocurable resin composition for a nail or an artificial nail according to claim 1, further comprising, as a component (E), a compound having a (meth)acryloyl group other than the component (A) and the component (B).

6. The photocurable resin composition for a nail or an artificial nail according to claim 1, wherein a glossiness of a surface of a cured product when the photocurable resin composition for a nail or an artificial nail is applied so as to have a film thickness of 0.1 mm and cured at an integrated light quantity of 7.5 kJ/m$^2$ and an uncured product on the surface of the cured product is wiped off with a solvent is 67 or more and 88 or less at 20°.

7. The photocurable resin composition for a nail or an artificial nail according to claim 1, wherein the photocurable resin composition for a nail or an artificial nail is for forming a topcoat layer.

8. A cured product obtained by curing the photocurable resin composition for a nail or an artificial nail according to any one of claims 1 to 6.

9. A method for coating a nail or an artificial nail, comprising applying the photocurable resin composition for a nail or an artificial nail according to any one of claims 1 to 6 to a nail or an artificial nail to form a coating film, and then irradiating the coating film with an energy ray to cure the coating film.

## INTERNATIONAL SEARCH REPORT

International application No.

**PCT/JP2022/022432**

### A. CLASSIFICATION OF SUBJECT MATTER

***A61K 8/87***(2006.01)i; ***A61K 8/35***(2006.01)i; ***A61K 8/37***(2006.01)i; ***A61K 8/46***(2006.01)i; ***A61K 8/55***(2006.01)i; ***A61K 8/81***(2006.01)i; ***A61Q 3/02***(2006.01)i

FI: A61K8/87; A61Q3/02; A61K8/81; A61K8/46; A61K8/37; A61K8/55; A61K8/35

According to International Patent Classification (IPC) or to both national classification and IPC

### B. FIELDS SEARCHED

Minimum documentation searched (classification system followed by classification symbols)

A61K8/87; A61K8/35; A61K8/37; A61K8/46; A61K8/55; A61K8/81; A61Q3/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2011/024810 A1 (BRIDGESTONE CORP.) 03 March 2011 (2011-03-03) claims, paragraph [0020], examples 1, 2, 4-6 | 1-3, 5-8 |
| Y | | 1-9 |
| X | JP 2017-203023 A (PRIOR CO., LTD.) 16 November 2017 (2017-11-16) claims, paragraphs [0003], [0005], [0028], [0029], examples 2, 3 | 1-3, 5-9 |
| Y | | 1-9 |
| Y | JP 2011-121867 A (THREE BOND CO., LTD.) 23 June 2011 (2011-06-23) paragraphs [0013], [0014] | 4, 8-9 |
| Y | JP 2008-174743 A (CRAY VALLEY S. A.) 31 July 2008 (2008-07-31) paragraphs [0001], [0014], [0018]-[0025] | 4, 8-9 |
| Y | JP 2005-289991 A (L'OREAL) 20 October 2005 (2005-10-20) claims 1-3, 69 | 4, 8-9 |

✓ Further documents are listed in the continuation of Box C. ✓ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **13 July 2022** | **02 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/022432**

C.    DOCUMENTS CONSIDERED TO BE RELEVANT

| Category* | Citation of document, with indication, where appropriate, of the relevant passages | Relevant to claim No. |
|---|---|---|
| Y | JP 2014-005260 A (SATODA KAKO KK) 16 January 2014 (2014-01-16)<br>claims, paragraphs [0030]-[0032], examples 2-7, 18, 20, 26, 27 | 1-9 |
| Y | WO 2016/072353 A1 (THREE BOND CO., LTD.) 12 May 2016 (2016-05-12)<br>claims, paragraphs [0022]-[0025] | 1-9 |
| Y | JP 2017-210475 A (SAKURA COLOR PRODUCTS CORP.) 30 November 2017<br>(2017-11-30)<br>claims, paragraph [0009], example 8 | 1-9 |

Form PCT/ISA/210 (second sheet) (January 2015)

**INTERNATIONAL SEARCH REPORT**
Information on patent family members

International application No.

**PCT/JP2022/022432**

| Patent document cited in search report | | | Publication date (day/month/year) | Patent family member(s) | | | Publication date (day/month/year) |
|---|---|---|---|---|---|---|---|
| WO | 2011/024810 | A1 | 03 March 2011 | US 2012/0157564 A1 claims, paragraph [0042], examples 1, 2, 4-6 EP 2471873 A1 CN 102482502 A | | | |
| JP | 2017-203023 | A | 16 November 2017 | US 2017/0319461 A1 claims, paragraphs [0004], [0006], [0029], [0030], experimental examples 2, 3 CN 107412009 A | | | |
| JP | 2011-121867 | A | 23 June 2011 | US 2012/0276028 A1 paragraphs [0019], [0020] WO 2011/071029 A1 EP 2510920 A1 CN 102770119 A KR 10-2012-0103682 A | | | |
| JP | 2008-174743 | A | 31 July 2008 | US 2008/0153924 A1 paragraphs [0002], [0028], [0034]-[0043] WO 2008/080536 A1 EP 1935934 A1 CN 101568581 A KR 10-2009-0103884 A | | | |
| JP | 2005-289991 | A | 20 October 2005 | US 2005/0220731 A1 claims 1-3, 81 EP 1595524 A1 | | | |
| JP | 2014-005260 | A | 16 January 2014 | (Family: none) | | | |
| WO | 2016/072353 | A1 | 12 May 2016 | US 2017/0312201 A1 claims, paragraphs [0030]-[0033] CN 107072924 A | | | |
| JP | 2017-210475 | A | 30 November 2017 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019006689 A
- JP 2017210475 A
- JP 2021095549 A